# EUROPEAN PATENT SPECIFICATION

(11) **EP 1 387 693 B1**
(45) Date of publication and mention of the grant of the patent: **09.08.2006**
(21) Application number: 02720307.4
(22) Date of filing: 03.05.2002
(51) Int. Cl.: A61K 39/02, A61P 31/04

(54) **SAPONIN INACTIVATED MYCOPLASMA VACCINE**
SAPONIN INAKTIVIERTE MYKOPLASMA IMPFSTOFFE
VACCIN

(30) Priority: 03.05.2001 GB 0110851
(43) Date of publication of application: 11.02.2004
(73) Proprietor: Novartis AG, 4056 Basel (CH)
(72) Inventor: NICHOLAS, Robin Ashley John, Vet.Labs Agency, Surrey, Kent KT15 3NB (GB)
(74) Representative: de Weerd, Petrus G.W.
(86) International application number: PCT/GB2002/002062
(87) International publication number: WO 2002/089838

(56) References cited:
- WO-A-01/34189
- WO-A-92/18161
- TOLA S ET AL: "Experimental vaccination against Mycoplasma agalactiae using different inactivated vaccines" VACCINE, BUTTERWORTH SCIENTIFIC. GUILDFORD, GB, vol. 17, no. 22, 16 July 1999 (1999-07-16), pages 2764-2768, XP004171707 ISSN: 0264-410X
- RURANGIRWA F R ET AL: "An inactivated vaccine for contagious caprine pleuropneumonia." THE VETERINARY RECORD. ENGLAND 24 OCT 1987, vol. 121, no. 17, 24 October 1987 (1987-10-24), pages 397-400, XP008008668 ISSN: 0042-4900
- NICHOLAS, R.A.J. ET AL: "An experimental vaccine for calf pneumonia caused by Mycoplasma bovis: clinical, cultural, serological and pathological findings"" VACCINE, vol. 20, 2002, pages 3569-3575,
- DE LA FE, C. ET AL: "Inactivation of Mycoplasma species involved in contagious agalactia" BERL. MÜNCH. TIERÄRZTL. WSCHR., vol. 117, 2004, pages 1-5,
- R. ROSENBUSCH: "Test of an inactivated vaccine against Mycoplasma bovis respiratory disease by transthoracic challenge with an abscessing strain" 1998, Proceedings of 12th Congress of the International Organisation of Mycoplasmology, Sydney. Page 185

## Description

The present invention relates to an inactivated strain of *Mycoplasma bovis,* which is a causative agent of bovine pneumonias, which is useful as a vaccine in cattle, to methods of producing it and to its use.

The present invention particularly relates to an inactivated strain of *Mycoplasma bovis.*

Mycoplasmas are microorganisms deprived of cell walls and are, therefore, a) pleomorphic and b) resistant to antibiotics of the beta-lactamine group, such as penicillin. Mycoplasmas vary considerably in their composition of their cell membrane and in particular in the quantity of cholesterol incorporated therein. The membrane of *M. mycoides* SC is surrounded by a carbohydrate layer (galactan). In addition, Mycoplasmas have inherently high mutation rates and so may rapidly develop systems capable of combating inactivation (e.g. production of enzymes etc) as has been seen in their response to antibiotics where resistance has developed rapidly.

*Mycoplasma bovis* is a primary cause of calf pneumonia, arthritis, mastitis, eye disease and other conditions worldwide and has been estimated to cost the cattle industry millions of pounds in mortality and setback losses annually. Calf pneumonia is a complex syndrome involving bacteria, viruses and mycoplasmas. In Europe it is believed that between 25-33% of calf pneumonias involve *Mycoplasma bovis.*

Recent strains *of Mycoplasma bovis* are showing increased resistance to a range of antibiotics (Ayling R et al (2000) Veterinary record 146, 745-747) and there is, therefore, a need for an effective vaccine.

Vaccines derived from inactivated strains of viruses or bacteria are well known in the art. The pathogenic microorganism is treated with a chemical, such as formaldehyde, which damages the organism to such an extent that it cannot cause infective disease in the animal to which it is administered. For example, the ability of the organism to replicate may be irreparably damaged. However, key antigenic regions of the organisms are retained, which stimulate an immune response in the animal. This response may provide a protective effect if the animal is later challenged with a potent strain of the organism.

Different inactivating agents have different modes of action in order to achieve the desired effect. Some agents bind DNA, others destroy or inactivate essential proteins and a further class of inactivating agents may disrupt the cell membrane. The efficacy of the agents in any particular case will depend upon the nature of the microorganism. Thus, the results achieved will be variable, as a result of the inherent variability of the microorganisms.

Rurangirwa et al. Veterinary Record (1987) 121,397-402 and Tola et al., Vaccine 17 (1999) 2764-2768 describe experimental vaccines based upon inactivated strains of F3 8 mycoplasma and *Mycoplasma agalactiae* respectively. WO01/34189, which was not published at the priority date of the present application, and so falls under Art 54(3) EPC, describes vaccines for *Mycoplasma bovis.*

It is not obvious that the use of a particular inactivating agent will produce the desired result in any particular instance. This is especially the case in different species where the response to a particular inactivating agent can be quite different. This is particularly true in the case of complex organisms such as *Mycoplasma bovis.*

An experimental quadrivalent vaccine incorporating formalin killed antigens of *Mycoplasma bovis* and *M dispar* together with viral and bacterial components was trialled (Howard et al (1987) Veterinary Record 121, 372-376. The vaccine included Quil A as adjuvant, and although effective, it was not successful enough to be produced commercially. Formalin inactivated vaccines incorporating *M bovis* and *Mannheima haemolytica* (Urbaneck et al (2000) Der Praktische Tierarzt 81, 756-763) and one incorporating two strains of *M. bovis* (Rosenbusch R (1998) Proceedings of 12^{th} Congress of the International Organisation of Mycoplasmology, Sydney p185) have also been reported with mixed success. A formalised *M bovis* vaccine against calf arthritis incorporating saponin as an adjuvant was immunogenic and reduced spread of infection to other joints in a small number of calves (Poumarat F et al (1999) Proceedings of International Symposium of Ruminant Mycoplasmas, Toulouse, June 1999 p65).

GB 1 074 920 is concerned with a vaccine for a pleuropneumonia-like organism (PPLO), which is suitable for human use, and a growth media for *Mycoplasma pneumoniae.* In this case the *Mycoplasma pneumoniae* is incubated in a sterile medium including evaporated chloroform extract of chicken egg yolk until antibody-inducing activity is imparted thereto, inactivated by use of formalin and concentrated. However, such formalised vaccines are not generally seen as effective. In addition, the vaccine described is for a specific human Mycoplasma and would not be sufficiently protective against bovine pneumonias in animals.

A number of attempts have been made to formulate a vaccine for *Mycoplasmas,* which are the causative agent for bovine pneumonia. None has, however, been particularly successful.

The applicants have surprisingly found, however, that by inactivating a strain of *Mycoplasma bovis,* which is a causative agent of bovine pneumonias with saponin, particularly effective vaccines may be produced.

According to the present invention, there is provided a strain of *Mycoplasma bovis,* which is a causative agent of bovine pneumonias, which has been inactivated by treatment with saponin.

When saponin is applied, the cell membrane is preferably only partially disrupted and is not totally disrupted. In this way, the cells are prevented from growing yet retain the key antigenic regions of the organisms, which stimulate an immune response in the animal.

Unlike the production of some other vaccines, which may include the step of lyophilisation, it is not necessary to lyophilise the Mycoplasma in order to produce the vaccine according to the present invention.

As used herein, the term "saponin" refers to sapogenin glycosides, which may be derived from plants such as Quillaga bark. A range of saponins containing various amounts of sapogenin are available from Sigma.

These compounds are known to have hemolytic activity and may be used as adjuvants for vaccines.

The strain of *Mycoplasma,* which is a causative agent of bovine pneumonias is a strain of *Mycoplasma bovis.* In this regard, an inactivated strain of *M. bovis* is used in vaccines to protect against pneumonias caused by *M. bovis.* Strains *of Mycoplasma bovis* are available from a variety of sources. However, suitable strains may be isolated from cattle, in particular from the lungs of cattle who have suffered from pneumonia. *Mycoplasma bovis* strain 96B/96 used in the present application was isolated from the lungs of a calf who had died of pneumonia.

Strains of the invention may be used in vaccines.

An advantage of the present invention is that it is surprisingly a highly effective vaccine. In addition, as the vaccines of the present invention are inactivated, they are inherently safer and more stable than live vaccine products.

Thus, a further aspect of the invention comprises a veterinary composition comprising an inactivated strain of *Mycoplasma bovis,* which is a causative agent of bovine pneumonias as described above, in combination with a veterinarily acceptable carrier.

The carrier may be solid or liquid, depending upon the intended mode of administration of the vaccine. Generally, however, the vaccine will be intended for parenteral administration. Liquid carriers, such as sterile aqueous or oily carriers for intravenous, subcutaneous, or intramuscular dosing may, therefore, be preferred.

Thus, the veterinary compositions of the present invention are suitably in the form of a sterile injectable aqueous or oily suspension, which may be formulated according to known procedures using one or more of the appropriate dispersing or wetting agents and suspending agents. A sterile injectable preparation may also be a sterile injectable solution or suspension in a non-toxic parenterally-acceptable diluent, for example a suspension in 1,3-butanediol.

Examples of suspending agents which may be used in the formulation of the invention include sodium carboxymethylcellulose, methylcellulose, hydroxypropylmethylcellulose, sodium alginate, polyvinyl-pyrrolidone, gum tragacanth and gum acacia. The aqueous suspensions may also contain one or more preservatives (such as ethyl or propyl p-hydroxybenzoate or anti-oxidants (such as ascorbic acid).

Preferably, the veterinary composition comprises only a strain of Mycoplasma according to the present invention and saponin in a buffered saline solution.

Oily suspensions may be formulated by suspending the active ingredient in a vegetable oil (such as arachis oil, olive oil, sesame oil or coconut oil) or in a mineral oil (such as liquid paraffin).

Dispersible powders and granules suitable for preparation of an aqueous suspension by the addition of water generally contain the active ingredient together with a dispersing or wetting agent, suspending agent and one or more preservatives.

The amount of active ingredient that is combined with one or more excipients to produce a single dosage form will necessarily vary depending upon the host treated and the particular route of administration. For example, a liquid formulation intended for subcutaneous administration to cattle and particularly calves, will generally contain, for example, from 0.1 mg to 0.5mg/ml of a strain of inactivated *Mycoplasma bovis* which is a causative agent of bovine pneumonias, compounded with an appropriate and convenient amount of excipients. Dosage unit forms will generally contain about 0.1 mg to about 2mg, suitably from about 0.2mg to 1mg of a strain of inactivated *Mycoplasma,* which is a causative agent of bovine pneumonias strain. In this regard, an inactivated strain of *M. bovis* is used in vaccines to protect against calf pneumonia found in Europe.

The size of the dose for prophylactic purposes will naturally vary according to the nature, the age of the animal and the route of administration, according to well known principles of medicine. Generally speaking however, the composition will be administered to young calves.

The composition of the invention may comprise further components, which would be known in the art. They may, for example include adjuvants such as aluminium compounds or Freund's incomplete adjuvant. Saponin, when present in the composition as a result of its use as an inactivating agent, will, however, also have an adjuvant effect, and it is unlikely, therefore, that further adjuvants would be required.

Where present, the saponin will be suitably be present in a liquid composition of the invention in an amount of at least 1mg/ml in order to produce an adjuvant effect. Preferably it will be present in an amount of from about 1-10mg/ml, and more suitably at about 2mg/ml.

In a further aspect, the invention provides a method of preparing an inactivated strain of *Mycoplasma bovis,* which is a causative agent of bovine pneumonias as described above, which method comprises incubating a strain of *Mycoplasma bovis,* which is a causative agent of bovine pneumonias in the presence of as saponin.

The amount of cell membrane disrupting agent present during the incubation should be sufficient to inactivate the strain and this will therefore depend to a large extent on the concentration of the strain within the culture, the precise nature of the strain, the particular agent used etc. However, where saponin is the agent, it should in general be present in the incubation culture in an amount of from about 0.5-10mg/ml, preferably at about 2mg/ml.

Once treated in this way, the inactivated strain may be isolated and formulated as discussed above. However, the direct product may be used as a vaccine formulation, provided all the components are veterinarily acceptable.

Where this is the case, saponin is present in the formulation and may exert an adjuvant effect.

Therefore, prevention of disease in cattle caused by a strain of *Mycoplasma bovis,* which is a causative agent of bovine pneumonias, can be achieved by administering to the cattle a veterinary composition as described above.
Suitably, the composition will be administered as a single dose, but booster doses may be administered if required or necessary, and these may be determined by usual clinical methods. For parenteral administration, dosage units as described above may be used.

The invention will now be particularly described by way of example.

### Example 1

### Preparation of Mycoplasma bovis Vaccine

Eaton's broth medium was prepared by dissolving Difco PPLO broth base (21 g) (without crystal violet) in 700mls of distilled water. Freshly prepared yeast extract (100mls) was added together with unheated horse serum (200mls), glucose (10g), and penicilin (0.5mls of 200,000 IU/ml), 0.2% phenol red (12.5mls) and 0.02% of DNA. The pH was adjusted to 7.6-7.8 and sterilised by fitration

A universal container with a frozen aliquot of the master seed preparation (10^{th} passage *of Mycoplasma bovis* strain 86B/96 isolated from the lungs of a calf which had died of pneumonia), kept in 10ml volumes at -70°C, was thawed completely in warm water.

This was added to 90mls of Eaton's broth medium, prepared as described above, and incubated for 48 hours at 38.5°C. This was subcultured into a 900 ml volume of Eaton's medium and incubated at 38.5°C for 48 hours. Cells were centrifuged at 10,000g for 30 minutes and resuspended in the same volume of phosphate buffered saline (PBS pH 7.2). The cells were centrifuged as before, resuspended in 1/10th of the original volume in PBS and adjusted to contain approximately 0.5mg/ml (approximately 10⁸ colony forming units per ml).

The mycoplasma cells were inactivated with saponin at 2mg/ml overnight at 37°C. The suspension was aliquoted into 20ml volumes and stored at 4°C.

### Example 2

### Biological testing

The vaccine (2ml), prepared as described in Example 1, was inoculated subcutaneously into each calf of four groups of seven or eight calves at about 4 weeks of age.

The four groups of cattle were housed separately.
- Group A (vaccinated/ challenged) containing 7 calves was inoculated with 2 mls of vaccine subcutaneously and challenged 3 weeks later with aerosol administration of virulent *M. bovis* (strain 5063) on consecutive days
- Group B (unvaccinated/challenged) containing 7 calves were injected with placebo and then challenged as for Group A
- Group C (unvaccinated/ not challenged controls) containing 8 calves were kept as uninoculated controls
- Group D **(vaccinated/not challenged)** containing 7 calves were immunised as for Group A but not challenged. These were monitored for adverse effects and antibody response

The vaccine was shown to be safe for calves and did not cause adverse clinical effects in groups A and D. It produced a strong serological responses in groups A and D within 2-3 weeks of administration. All calves in group B developed clinical signs of pneumonia while group A showed fewer signs. There was a significant decrease in body weight gain in group B compared to group A and a significant increase in lung lesions and rectal temperatures in group B calves.

In conclusion the *M. bovis* vaccine produced a significant level of protection against a virulent challenge.

## Claims

1. A strain *of Mycoplasma bovis* which is a causative agent of bovine pneumonias and which has been in activated by treatment with saponin.

2. A veterinary composition comprising an inactivated strain of *Mycoplasma bovis* as claimed in claim 1, in combination with a veterinarily acceptable carrier.

3. A composition according to claim 2, which is suitable for parenteral administration.

4. A composition according to claim 2 or claim 3 wherein the veterinarily acceptable carrier is a liquid carrier.

5. A composition according to any one of claims 2 to 4 which further comprises one or more of a dispersing agent, a wetting agent or a suspending agent.

6. A composition according to any one of claims 2 to 5, which further comprises an adjuvant.

7. A composition according to claim 6 wherein the adjuvant is saponin.

8. A method of preparing an inactivated strain of *Mycoplasma bovis,* which is a causative agent of bovine pneumonias, which method comprises incubating a strain of *Mycoplasma bovis* which is a causative agent of bovine pneumonias in the presence of saponin.

9. A method according to claim 8 wherein the saponin is present in the incubation culture in an amount of from about 0.5-10mg/ml.

10. A method according to claim 8 wherein the saponin is present in an amount of about 2mg/ml.

11. The use of a strain of *Mycoplasma bovis* according to claim 1, in the preparation of a medicament for the treatment of animals.

12. The use according to claim 11 wherein the medicament is for the prophylaxis of bovine pneumonias caused by a strain of *Mycoplasma bovis.*

## Patentansprüche

1. Stamm von *Mycoplasma bovis,* der einen ursächlichen Erreger von Rinderpneumonien darstellt und der durch Behandlung mit Saponin inaktiviert wurde.

2. Veterinäre Zusammensetzung, umfassend einen inaktivierten Stamm von *Mycoplasma bovis* nach Anspruch 1, in Kombination mit einem veterinär verträglichen Träger.

3. Zusammensetzung nach Anspruch 2, die zur parenteralen Verabreichung geeignet ist.

4. Zusammensetzung nach Anspruch 2 oder Anspruch 3, worin der veterinär verträgliche Träger ein flüssiger Träger ist.

5. Zusammensetzung nach einem der Ansprüche 2 bis 4, die weiterhin eines oder mehrere von einem dispergierenden Mittel, einem Benetzungsmittel oder einem Suspendiermittel umfasst.

6. Zusammensetzung nach einem der Ansprüche 2 bis 5, die weiterhin ein Hilfsmittel umfasst.

7. Zusammensetzung nach Anspruch 6, worin das Hilfsmittel Saponin ist.

8. Verfahren zur Herstellung eines inaktivierten Stamms von *Mycoplasma bovis,* der einen ursächlichen Erreger von Rinderpneumonien darstellt, wobei das Verfahren Inkubieren eines Stamms von *Mycoplasma bovis,* der einen ursächlichen Erreger von Rinderpneumonien darstellt, in Gegenwart von Saponin umfasst.

9. Verfahren nach Anspruch 8, wobei das Saponin in der Inkubationskultur in einer Menge von etwa 0,5-10 mg/ml vorliegt.

10. Verfahren nach Anspruch 8, wobei das Saponin in einer Menge von etwa 2 mg/ml vorliegt.

11. Verwendung eines Stamms von *Mycoplasma bovis* nach Anspruch 1 zur Herstellung eines Arzneimittels für die Behandlung von Lebewesen.

12. Verwendung nach Anspruch 11, wobei das Arzneimittel zur Prophylaxe von Rinderpneumonien, die durch einen Stamm von *Mycoplasma bovis* verursacht werden, vorgesehen ist.

## Revendications

1. Souche de *Mycoplasma bovis* qui est un agent causal de pneumonies bovines et qui a été inactivée par traitement à la saponine.

2. Composition vétérinaire comprenant une souche inactivée de *Mycoplasma bovis* telle que revendiquée dans la revendication 1, en combinaison avec un véhicule acceptable d'un point de vue vétérinaire.

3. Composition selon la revendication 2, qui convient pour l'administration parentérale.

4. Composition selon la revendication 2 ou 3, dans laquelle le véhicule acceptable d'un point de vue vétérinaire est un véhicule liquide.

5. Composition selon l'une quelconque des revendications 2 à 4, qui comprend en outre un ou plusieurs agents parmi un agent dispersant, un agent mouillant ou un agent de suspension.

6. Composition selon l'une quelconque des revendications 2 à 5, qui comprend en outre un adjuvant.

7. Composition selon la revendication 6, dans laquelle l'adjuvant est la saponine.

8. Procédé de préparation d'une souche inactivée de *Mycoplasma bovis,* qui est un agent causal de pneumonies bovines, ledit procédé comprenant l'incubation d'une souche de *Mycoplasma bovis* qui est un agent causal de pneumonies bovines en présence de saponine.

9. Procédé selon la revendication 8, dans lequel la saponine est présente dans la culture d'incubation en une quantité d'environ 0,5-10 mg/ml.

10. Procédé selon la revendication 8, dans lequel la saponine est présente en une quantité d'environ 2 mg/ml.

11. Utilisation d'une souche de *Mycoplasma bovis* selon la revendication 1, dans la préparation d'un médicament destiné au traitement d'animaux.

12. Utilisation selon la revendication 11, dans laquelle le médicament est destiné à la prophylaxie de pneumonies bovines causées par une souche de *Mycoplasma bovis.*
